# EUROPEAN PATENT SPECIFICATION

(11) **EP 4 076 222 B1**
(45) Date of publication and mention of the grant of the patent: **22.05.2024**
(21) Application number: 20838987.4
(22) Date of filing: 18.12.2020
(51) Int. Cl.: A61B 17/22

(54) **VIBRATIONAL WIRE GUIDE FOR CORING AND ASPIRATING A VENOUS OBSTRUCTION**
VIBRATIONSDRAHTFÜHRUNG ZUM AUSSTANZEN UND ABSAUGEN EINER VENÖSEN VERSTOPFUNG
GUIDE DE FIL VIBRATOIRE POUR LE PERCAGE ET L'ASPIRATION D'UNE OBSTRUCTION VEINEUSE

(30) Priority: 20.12.2019 US 201962951351 P
(43) Date of publication of application: 26.10.2022
(73) Proprietor: Koninklijke Philips N.V., 5656 AG Eindhoven (NL)
(72) Inventor: GUNDERSON, Richard, Charles, 5656 AE Eindhoven (NL); EKELUND, Connor, 5656 AE Eindhoven (NL)
(74) Representative: Philips Intellectual Property & Standards
(86) International application number: PCT/EP2020/087342
(87) International publication number: WO 2021/123395

(56) References cited:
- US-A- 4 936 845
- US-A- 5 423 846
- US-A1- 2006 293 612
- US-A1- 2015 057 694

## Description

### FIELD

The present disclosure relates generally to medical devices and the use of medical devices for the treatment of vascular conditions. In particular, the present disclosure provides devices for using a vibrational wire guide to cut and/or core through a venous obstruction, such as a chronic clot.

### BACKGROUND

Peripheral vascular disease refers to diseased blood vessels in a subject's vascular system away from the subject's heart and brain. Although peripheral vascular disease can occur within a subject's arteries (arterial system) or veins (venous system), peripheral vascular disease typically occurs in a subject's venous system and often in the legs.

Veins return blood to the heart from all the body's organs. To reach the heart, the blood needs to flow upward from the veins in the legs. Calf muscles and the muscles in the feet need to contract with each step to squeeze the veins and push the blood upward. To keep the blood flowing up, and not back down, the veins contain one-way valves.

Venous insufficiency occurs when these valves become damaged, allowing the blood to leak backward. Valve damage may occur as the result of aging, extended sitting or standing or a combination of aging and reduced mobility. When the veins and valves are weakened to the point where it is difficult for the blood to flow up to the heart, blood pressure in the veins stays elevated for long periods of time, leading to thrombosis.

Thrombosis is the formation of a blood clot, known as a thrombus, within a blood vessel. It prevents blood from flowing normally through the circulatory system. When a blood clot forms in the veins, it is known as venous thromboembolism. This can cause deep vein thrombosis (DVT).

There are three primary classifications of DVTs, and they are based on how long the blood clot is present. When a blood clot forms and has been around for 14 days or less, then it is called acute DVT. The blood clot in a DVT doesn't get very hard or become tightly attached to the walls of the vein during this short period of time, thereby making the acute DVT relatively easier to treat than the other types of DVTs. For example, an acute DVT caught early enough can be treated with clot-dissolving medications.

The second classification of DVTs is called a subacute DVT, which is when the blood clot exists for between 14 to 28 days. The blood clot in the subacute stage is likely to have become slightly harder than it was during the acute stage, but not as hard as it will get in the chronic stage.

The third main classification is a chronic DVT, which is when the blood clot persists for 28 days or longer. In this instance, the blood clot in the subject's arm or leg has had the chance to harden and form connections with the walls of the vein, which will later become scarred tissue inside of the vein.

When a blood clot persists for longer than about 28 days, its composition changes from primarily fibrinous (capable of dissolving via natural lytics) to crosslinked collagen. As a thrombus matures it undergoes reorganization in a process that resembles wound healing. Leukocytes and other inflammatory cells infiltrate the thrombus; cellular components are replaced by collagen deposits, and a neovascular network is formed. These processes alter the composition and properties of the thrombus, provoking its resistance to thrombolytic therapies. The hardening of chronic thrombi is due in large part to the cross linking of fibrin and replacement of cellular material by collagen. For example, by 1 week, thrombus collagen content may reach approximately 20%, and after three weeks it may be as high as 80%.

Crosslinked collagen is rubbery, elastic and tough, and a chronic clot can attach strongly to the vein wall via tendrils called synechiae. Once developed, a chronic clot can drastically reduce venous blood flow, causing significant negative symptoms. Conventional interventional techniques like angioplasty, debulking, and stenting are not efficacious in the treatment of the crosslinked collagen in chronic clots due to the difficulty of removal of the clots without disrupting the vein walls to which they are attached. Additionally, in order to treat chronic clots conventional interventional techniques it is often necessary to cross the clot with a guidewire in order to introduce the interventional devices. But due to the hardening of the chronic clot, known guidewires are unable to penetrate and cross the clot.

US 4936845 A relates to a drive catheter having a distal tip for opening a partially or totally obstructed blood vessel. Both manual and motor applied motions are disclosed. In addition to a continuous rotation, two reciprocating motions are disclosed. One reciprocating motion is a back and forth translation of the tip in a ramming motion. A second reciprocating motion is a back and forth rotation about an axis. Different shaped tips are disclosed for the two reciprocating motions.

US 2015/057694 A1 relates to a method of penetrating an intravascular occlusion including the steps of: obtaining an apparatus with a sleeve assembly defining an elongate passageway having a lengthwise axis, a stabilizing assembly, and a blade assembly; directing the sleeve assembly into an operative position within a vessel passage wherein an outlet from the elongate passageway is adjacent an occlusion within the vessel passage; through the stabilizing assembly, stabilizing the sleeve assembly in the operative position; and with the sleeve assembly stabilized in its operative position advancing the blade assembly in a first lengthwise direction relative to the sleeve assembly to penetrate the occlusion.

### SUMMARY

The object of the present invention is solved by the subject-matter of the independent claims; further embodiments are incorporated in the dependent claims. Methods as such do not form part of the claimed subject matter. There are certain rotatable cutting devices that may be used to cut through and/or core certain vascular lesions. But it may not be desirable to use a rotatable cutting device for cutting through and/or coring a chronic clot. What is needed is a non-rotatable cutting and/or coring device to penetrate and cross the clot in order to introduce a guidewire. The present disclosure discusses a non-rotating wire guide that penetrates the chronic clot by ultrasound vibrational movement. These and other needs are addressed by the various aspects, embodiments, and configurations of the present disclosure. For example, the present disclosure discusses a wire guide that has a specially designed non-rotatable head for cutting and coring through a chronic clot, thereby creating a passageway for inserting a wire guide therethrough.

An example of a method for creating a lumen through an obstruction within the venous system within a subject of the present disclosure comprises locating an obstruction in the venous system of the subject, positioning a balloon catheter within the venous system of the subject adjacent the obstruction, wherein the balloon catheter comprises an expandable member and the expandable member is expanded within the venous system, positioning an aspiration catheter within the vasculature of the subject, wherein the aspiration catheter extends beyond a distal end of the balloon catheter, positioning a non-rotatable wire guide within the venous system of the subject, wherein the non-rotatable wire guide comprises a cutting head and the cutting head is adjacent the chronic clot, wherein the cutting head comprises a proximal end and a distal end, wherein the proximal end comprises a concave shape and the distal end comprises a convex shape, wherein the concave shape comprises a most proximal end and a most distal end, wherein the proximal end of the cutting head comprises a diameter, wherein a ratio of a length between the distal end of the cutting head and the most proximal end of the concave shape is relative to the diameter of the cutting head is between 1.5:1 and 3:1, introducing a fluid into the balloon catheter or the aspiration catheter, and applying ultrasonic energy to the non-rotatable wire guide such that the cutting head of the non-rotatable wire guide translates back and forth axially without rotating, and aspirating the fluid during translation of the cutting head.

Another example includes the method of the preceding paragraph further comprising the step of re-positioning the cutting head.

Another example includes the method of any preceding paragraph further comprising the step of aspirating the fluid.

Another example includes the method of any preceding paragraph, wherein the cutting head comprise a plurality of blades evenly spaced around a circumference of the cutting head.

Another example includes the method of any preceding paragraph, wherein the blades are substantially parallel to and aligned with a longitudinal axis of the shaft.

Another example includes the method of any preceding paragraph, wherein the blades have a proximal end, a distal end, a height and a width.

Another example includes the method of any preceding paragraph, wherein the height of at least one of the plurality of blades increases from the distal end to the proximal end of the at least one of the plurality of blades.

Another example includes the method of any preceding paragraph, wherein the width of at least one of the plurality of blades increases from the distal end to the proximal end of the at least one of the plurality of blades.

An example of a catheter system of the present disclosure comprises a balloon catheter comprising an expandable member, an aspiration catheter configured to extend beyond a distal end of the balloon catheter, and a non-rotatable wire guide comprising a cutting head, wherein the cutting head comprises a proximal end and a distal end, wherein the proximal end comprises a concave shape and the distal end comprises a convex shape, wherein the concave shape comprises a most proximal end and a most distal end, wherein the proximal end of the cutting head comprises a diameter, wherein a ratio of a length between the distal end of the cutting head and the most proximal end of the concave shape relative to the diameter of the cutting head is between 1.5: 1 and 3: 1, wherein the non-rotatable wire guide such is configured to translates back and forth axially without rotating.

Another example includes the catheter system of the preceding paragraph, wherein the cutting head comprise a plurality of blades evenly spaced around a circumference of the cutting head.

Another example includes the catheter system of the preceding paragraphs, wherein the blades are substantially parallel to and aligned with a longitudinal axis of the shaft.

Another example includes the catheter system of the preceding paragraphs, wherein the blades have a proximal end, a distal end, a height and a width.

Another example includes the catheter system of the preceding paragraphs, wherein the height of at least one of the plurality of blades increases from the distal end to the proximal end of the at least one of the plurality of blades.

Another example includes the catheter system of the preceding paragraphs, wherein the width of at least one of the plurality of blades increases from the distal end to the proximal end of the at least one of the plurality of blades.

Another example includes the catheter system of the preceding paragraphs, wherein the width of at least one of the plurality of blades increases from the distal end to the proximal end of the at least one of the plurality of blades.

As used herein, "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C," and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (for example, X₁ and X₂) as well as a combination of elements selected from two or more classes (for example, Y₁ and Zₒ).

It is to be noted that the term "a" or "an" entity refers to one or more of that entity. As such, the terms "a" (or "an"), "one or more" and "at least one" can be used interchangeably herein. It is also to be noted that the terms "comprising," "including," and "having" can be used interchangeably.

The term "about" when used in conjunction with a numeric value shall mean plus and/or minus ten percent (10%) of that numeric value, unless otherwise specifically mentioned herein.

The term "catheter" as used herein generally refers to a tube that can be inserted into a body cavity, duct, lumen, or vessel, such as the vasculature system. In most uses, a catheter is a relatively thin, flexible tube ("soft" catheter), though in some uses, it may be a larger, solid, less flexible-but possibly still flexible-catheter ("hard" catheter). In some uses a catheter may contain a lumen along part or all of its length to allow the introduction of other catheters or guidewires. An example of a catheter is a sheath.

The term "balloon catheter" as used herein generally refers to the various types of catheters which carry a balloon for containing fluids. Balloon catheters may also be of a wide variety of inner structure, such as different lumen design, of which there are at least three basic types: triple lumen, dual lumen and co-axial lumen. All varieties of internal structure and design variation are meant to be included by use of the term "balloon catheter" herein. In some uses, balloon catheters can be used to perform angioplasty.

The term "means" as used herein shall be given its broadest possible interpretation in accordance with 35 U.S.C. § 112(f). Accordingly, a claim incorporating the term "means" shall cover all structures, materials, or acts set forth herein, and all of the equivalents thereof. Further, the structures, materials or acts and the equivalents thereof shall include all those described in the summary, brief description of the drawings, detailed description, abstract, and claims themselves.

The term "sheath" as used herein generally refers to a tube that can be inserted into a body cavity duct, lumen, or vessel, such as the vasculature system that allows for the introduction of other devices, such as catheters, and the introduction of fluid along its length. The sheath can have a closed end or an open end. Because the sheath is a tube that can be inserted into a body cavity, duct, lumen, or vessel, such as the vasculature system, the sheath may also be considered a catheter. Accordingly, a catheter, such as a laser catheter, can be introduced into another catheter.

The term "therapeutic agent" as used herein generally refers to any known or hereafter discovered pharmacologically active agent that provides therapy to a subject through the alleviation of one or more of the subject's physiological symptoms. A therapeutic agent may be a compound that occurs in nature, a chemically modified naturally occurring compound, or a compound that is chemically synthesized. The agent will typically be chosen from the generally recognized classes of pharmacologically active agents, including, but not necessarily limited to, the following: analgesic agents; anesthetic agents; antiarthritic agents; respiratory drugs, including antiasthmatic agents; anticancer agents, including antineoplastic drugs; anticholinergics; anticonvulsants; antidepressants; antidiabetic agents; antidiarrheals; antihelminthics; antihistamines; antihyperlipidemic agents; antihypertensive agents; anti-infective agents such as antibiotics and antiviral agents; antiinflammatory agents; antimigraine preparations; antinauseants; antiparkinsonism drugs; antipruritics; antipsychotics; antipyretics; antispasmodics; antitubercular agents; antiulcer agents; antiviral agents; anxiolytics; appetite suppressants; attention deficit disorder (ADD) and attention deficit hyperactivity disorder (ADHD) drugs; cardiovascular preparations including calcium channel blockers, CNS agents; beta-blockers and antiarrhythmic agents; central nervous system stimulants; cough and cold preparations, including decongestants; diuretics; genetic materials; herbal remedies; hormonolytics; hypnotics; hypoglycemic agents; immunosuppressive agents; leukotriene inhibitors; mitotic inhibitors; restenosis inhibitors; muscle relaxants; narcotic antagonists; nicotine; nutritional agents, such as vitamins, essential amino acids and fatty acids; ophthalmic drugs such as antiglaucoma agents; parasympatholytics; psychostimulants; sedatives; steroids; sympathomimetics; tranquilizers; and vasodilators including general coronary, peripheral and cerebral.

The terms "vasculature" and "vascular" as used herein refer to any part of the circulatory system of a subject, including peripheral and non-peripheral arteries and veins. Vasculature can be comprised of materials such as nucleic acids, amino acids, carbohydrates, polysaccharides, lipids fibrous tissue, calcium deposits, remnants of dead cells, cellular debris and the like.

The term "vascular occlusion" or "occlusion" refers to buildup of fats, lipids, fibrin, fibro-calcific plaque, thrombus and other atherosclerotic tissue within the lumen or within the intima of an artery that either narrows or completely obstructs the inner lumen the artery thereby restricting or blocking normal blood flow through the artery segment. The occlusion may partially or totally occlude the vasculature. Accordingly, the term "vascular occlusion" or "occlusion" shall include both a total occlusion and a partial occlusion. Alternatively, a vascular occlusion or occlusion may also be referred to as a vascular obstruction (or obstruction) or a vascular restriction (or restriction). A vascular obstruction may, therefore, be referred to as a total obstruction or a partial obstruction, and a vascular restriction may be referred to as a total restriction or a partial restriction.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 illustrates an exemplary system, including an ultrasonic generator and a non-rotatable wire guide that transmits and emits ultrasonic energy.
FIG. 2 is a side view of a kit that includes the non-rotatable wire guide, an aspiration catheter assembly and a balloon catheter assembly.
FIG. 3 is a side view of the balloon catheter assembly of FIG. 2.
FIG. 4 is a side view of the aspiration catheter assembly of FIG. 2.
FIG. 5 is an enlarged side view of the distal portions of the kit 500 within line 5-5 of FIG. 2, wherein a portion of the aspiration catheter assembly extends beyond the distal end of the balloon catheter assembly, and wherein a portion of the non-rotatable wire guide extends beyond the aspiration catheter assembly.
FIG. 6 is an enlarged side view of the cutting head of the non-rotatable wire guide illustrated in FIGs. 1 and 5.
FIG. 6A is a perspective view of the cutting head of the non-rotatable wire guide illustrated in FIG. 6.
FIG. 7A is an enlarged side view of an alternative cutting head of the non-rotatable wire guide illustrated in FIGs. 1 and 5.
FIG. 7B is a cross-sectional view of the alternative cutting head illustrated in FIG. 7A taken along line B-B.
FIG. 8A is a longitudinal-sectional view of a vein taken along a direction parallel to the longitudinal axis of the vein, wherein a chronic clot exists within the vein.
FIG. 8B is a longitudinal-sectional view of a balloon catheter adjacent the chronic clot in the vein.
FIG. 8C is a longitudinal-sectional view of an aspiration catheter extending distally of the balloon catheter adjacent the chronic clot in the vein.
FIG. 8D is a longitudinal-sectional view of a cutting head extending distally of the aspiration catheter and balloon catheter, wherein the cutting head is adjacent and proximal the chronic clot in the vein.
FIG. 8E is a longitudinal-sectional view of the cutting head extending distally of the aspiration catheter and balloon catheter, wherein the cutting head is extending into the chronic clot in the vein and coring a lumen through the chronic clot.
FIG. 8F is a longitudinal-sectional view of the cutting head extending distally of the aspiration catheter and balloon catheter, wherein the cutting head extends completely through the chronic clot in the vein after coring a lumen through the chronic clot.
FIG. 9 is a representative flow diagram of a method of treating a subject using the kit illustrated in FIG. 2.
FIG. 10 is an enlarged cross-sectional view of vein having a chronic clot with a lumen therethrough that was created by the non-rotatable wire guide(s) illustrated in FIGs. 5, 6, 7A and 7B using the method depicted in FIG. 9.

### DETAILED DESCRIPTION

The present disclosure relates generally to the use of medical devices for the treatment of vascular conditions. In particular, the present disclosure provides materials and systems for using laser-induced pressure waves to disrupt vascular blockages and to deliver therapeutic agents to the blockage area.

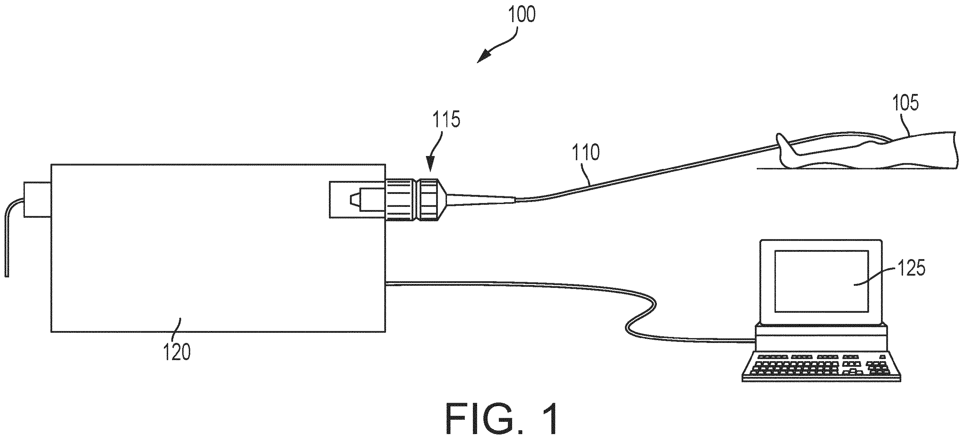

Referring to FIG. 1, there is depicted an exemplary ultrasonic system 100 of the present disclosure. Ultrasonic system 100 includes an ultrasonic generator 120 coupled to a controller 125. Controller 125 includes one or more computing devices programmed to control the ultrasonic generator 120. Controller 125 may be internal or external to the ultrasonic generator 120. In some embodiments, ultrasonic generator 120 produces ultrasonic energy in fixed or modulating pulses at a certain frequency or a variety of frequencies with a predetermined range.

The ultrasonic generator 120 is connected to the proximal end of a non-rotatable wire guide 110 via a coupler 115. The distal end of the non-rotatable wire guide 110 may be inserted into the vascular system or tissue of a human subject 105. For example, the distal end of the non-rotatable wire guide 110 may be inserted into the venous system of the subject through one or more types of catheters as discussed herein below.

The controller 125 of FIG. 1 includes a non-transitory computer-readable medium (for example, memory (not shown)) that includes instructions that, when executed, cause one or more processors (not shown) to control the ultrasonic generator 120 and/or other components of ultrasonic system 100. Controller 125 includes one or more input devices to receive input from an operator. Exemplary input devices include keys, buttons, touch screens, dials, switches, mouse, and trackballs which providing user control of ultrasonic generator 120. Controller 125 further includes one or more output devices to provide feedback or information to an operator. Exemplary output devices include a display, lights, audio devices which provide user feedback or information.

FIG. 1 depicts the non-rotatable wire guide 110 entering the human subject, preferably through the femoral vein or iliac vein or great saphenous vein or other vein in the leg of the human subject. As discussed above, it may be preferable to treat peripheral vascular disease, such as a thrombus or a chronic clot formed in the subject's venous system. Additionally, the ultrasonic system 100 may be used to treat either coronary arterial disease (CAD) or other type of peripheral arterial disease (PAD). If the ultrasonic system 100 is used to treat CAD, the non-rotatable wire guide 110 may enter the femoral artery, and the non-rotatable wire guide 110 will be directed through the patient's vasculature system and to the coronary arteries. Alternatively, if the wire guide 110 is intended to treat PAD, the non-rotatable wire guide 110 will be directed through the patient's vasculature system and to the peripheral arteries, such as the vasculature below the knee, particularly the vasculature in the patient's legs and/or feet.

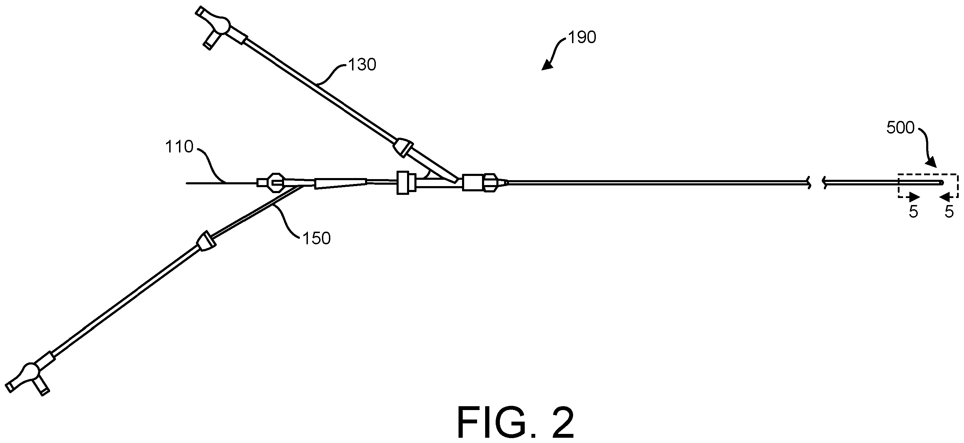

Referring to FIG. 2, there is depicted a kit 190 that includes a balloon catheter assembly 130 and an aspiration catheter assembly 150 and the non-rotatable wire guide 110. Balloon catheter assembly 130 may also be referred to as outer sheath assembly or outer catheter assembly 130 due to its disposition with respect to the aspiration catheter assembly 150 and non-rotatable wire guide 110.

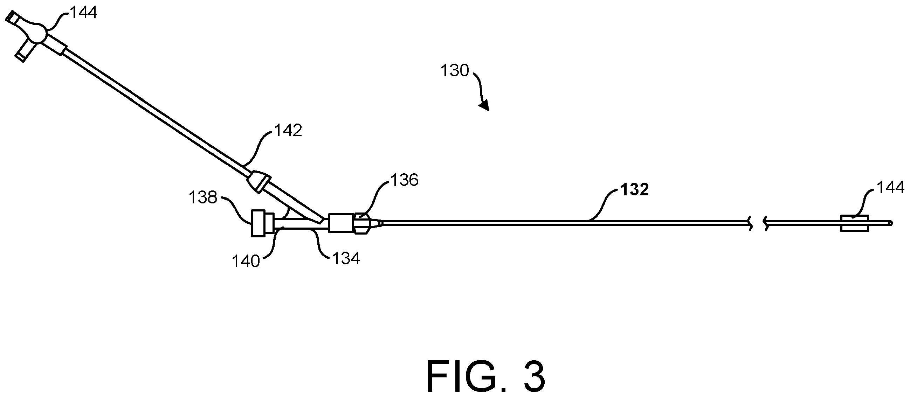

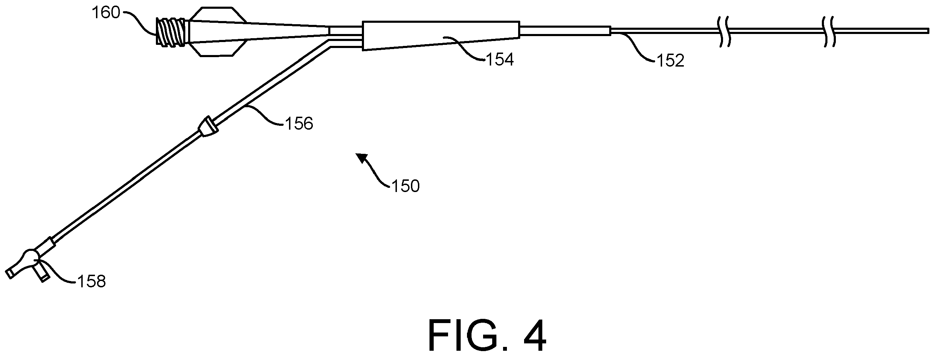

FIG. 3 depicts the balloon catheter assembly 130 shown in FIG. 2, and FIG. 4 depicts the aspiration catheter assembly 150 shown in FIG. 2. The balloon catheter assembly 130 may include a proximal end portion, a distal end portion and a catheter or sheath 132 having a working length of about between 50 cm and 200 cm, including 140 cm, and a lumen extending between such ends. The proximal end of the balloon catheter assembly 130 may include a bifurcate 134 (or Y connector) that is coupled to the sheath 130 by a Luer fitting 136. The bifurcate 134 may comprise homeostasis valve that includes a tube 140 extending in one direction (e.g., an axial direction) and another tube 142 that extends in a direction offset from tube 140. The tube 140 may have an opening 138 through which the aspiration catheter assembly 150 may enter. The tube 140 may also comprise a hemostasis valve at or adjacent the opening 138. The aspiration catheter assembly 150 is capable of extending from the proximal end of the balloon catheter assembly 130 to the distal end of the balloon catheter assembly 130 through a lumen therein. The tube 142 may include a stopcock 144 through which the liquid medium may enter the balloon assembly 130. The liquid medium may serve to inflate the inflatable member or serve as the irrigation fluid.

Referring to FIG. 4, there is depicted the aspiration catheter assembly 150 shown in FIG. 2. The aspiration catheter assembly 150 may include a proximal end portion, a distal end portion and a catheter or sheath 152 having a working length of about between 50 cm and 200 cm, including 140 cm, and a lumen extending between such ends. The proximal end of the aspiration catheter assembly 150 may include a bifurcate 154 (or Y connector) that is coupled to the sheath 152 by a Luer fitting 156. The bifurcate 154 may comprise a tube 150 extending in one direction (e.g., an axial direction) and another tube 152 that extends in a direction offset from tube 150. The tube 150 may have an opening 158 through which non-rotatable wire guide 110 may enter. The tube 150 may also comprise a hemostasis valve at or adjacent the opening 158. The non-rotatable wire guide 110 is capable of extending from the proximal end of the aspiration catheter assembly 150 to the distal end of the aspiration catheter assembly 150 through a lumen therein. The tube 152 may include a stopcock 154 through which the aspiration fluid may exit the aspiration catheter assembly 150.

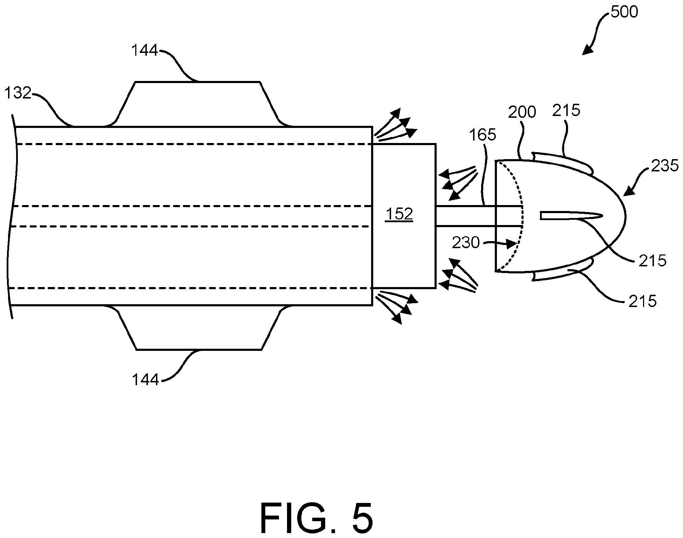

Referring to FIG. 5, there is depicted a distal portion 500 of the kit 190, particularly distal portions of the balloon catheter assembly 130, the aspiration catheter assembly 150 and the shaft 165 and cutting head 200 of the non-rotatable wire guide 110. As shown in this figure, the distal portion of the balloon catheter assembly 130 includes an expandable member 144 (e.g., balloon) surrounding the exterior of the sheath 132. The sheath 132 includes a lumen, and the distal portion of the aspiration catheter assembly 150, namely the sheath 152, extends from the sheath 132 of the balloon catheter assembly 130 through the lumen of the sheath 132. The arrows between the distal ends of the sheath 132 and 152 are indicative of irrigation fluid entering the vascular (e.g., venous) system between the distal ends of the sheaths 132, 152.

Continuing to refer to FIG. 5, there is depicted a distal portion of the non-rotatable wire guide 110 including a wire guide shaft 165 and a cutting or coring head 200 attached to its distal end. The sheath 152 includes a lumen, and the distal portion of the non-rotatable wire guide 110 (including cutting head 200) extends from the sheath 152 of the aspiration catheter assembly 150 through the lumen of the sheath 152. The arrows between the distal ends of the sheath 152 and shaft 165 of the non-rotatable wire guide 110 are indicative of aspirating fluid exiting the vascular system (e.g., venous system) between the distal ends of the sheath 152 and the non-rotatable wire guide 110.

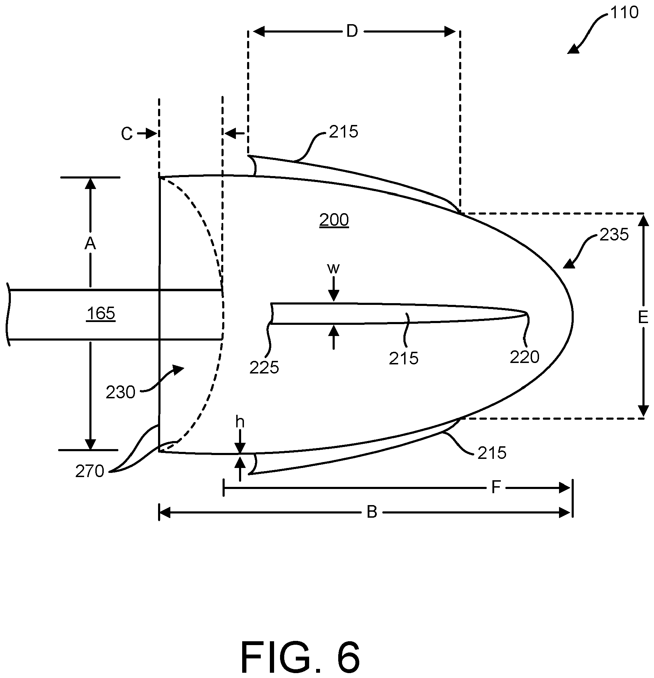

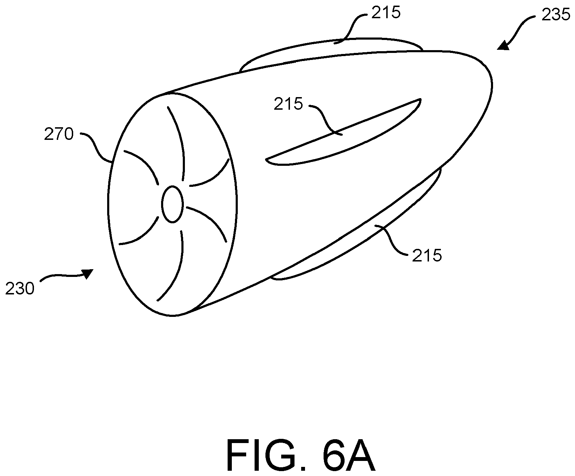

Referring to FIG. 6 and FIG. 6A, there is depicted an enlarged illustration of distal portion of the non-rotatable wire guide 110 including the wire guide shaft 165 and the cutting head 200. As shown in these figures, cutting head 200 includes a concave-shaped proximal end 230 and a convex-shaped distal end 235. Regarding the concave-shaped proximal end 230, this portion of the cutting head 200 has a cup or bowl shape because this end curves inwardly from the proximal end to the distal end of the cutting head 200 as this end also curves inwardly from the circumferential cutting edge 230 to the center of the cutting head 200, where the cutting head 200 is attached to the shaft 165.

The cutting head 200 also includes a plurality of blades 215 aligned with and/or parallel to the longitudinal axis of the non-rotatable wire guide 110, including the wire guide shaft 165 and the cutting head 200, between its proximal and distal ends. The plurality of blades 215 are also evenly spaced around the circumference of the cutting head 200 such that the blades 215 are substantially parallel to and aligned with the longitudinal axis of the shaft 165 of the wire guide 165 and cutting head 200. Each of the blades 215 has a height (h) extending from the surface of the cutting head 200. At the top of the height of each blade 215, there is a sharp surface to cut through the chronic clot or thrombus. The blades 215 also have a proximal end 225 and a distal end 220, and as such the blades 215 having a length (D). The height (h) of each or some of the blades 215 may be constant from the distal end 220 to the proximal end 225, the height (h) of each or some of the blades 215 may increase as the blade progresses from the distal end 220 to the proximal end 225, or the height (h) of each or some of the blades 215 may decrease as the blade progresses from the distal end 220 to the proximal end 225. The configuration(s) of the height of the blades 215 may increase the ability and effectiveness of the blades 215 to cut through the chronic clot or thrombus. The proximal end 230 of the concave portion of the cutting head 200 can have a cutting edge 235. Cutting edge 235 can aid in the coring of the lesion or chronic clot with the longitudinal movement or vibration of the wire guide 110 from the ultrasound frequency transmitted to the wire guide 110 via the system transducer. The cutting edge 230 is sharp and depicted as having a flat profile, but the cutting edge 230 may alternatively have a serrated or scalloped proximally facing profile. Because the cutting edge 230 is formed at the intersection of the circumferential exterior of the cutting head 200 and its concave portion, at least a portion of the cutting edge 230 is facing at proximally.

The width (w) of each or some of the blades 215 may be constant from the distal end 220 to the proximal end, the width (w) of each or some of the blades 215 may increase as the blade progresses from the distal end 220 to the proximal end 225, or the width (w) of each or some of the blades 215 may decrease as the blade progresses from the distal end 220 to the proximal end 225. The configuration(s) of the width of the blades 215 may increase the ability and effectiveness of the blades 215 to cut through the chronic clot or thrombus.

As mentioned above, the cutting head 200 includes a concave-shaped proximal end 230. The concave shape of the proximal end of the cutting head 200 aids in the aspiration of the irrigation fluid and clot debris as the non-rotatable wire guide 110 (including the wire guide shaft 165 and the cutting head 200) repeatedly oscillate axially back and forth along the longitudinal axis of the non-rotatable wire guide 110, thereby directing the irrigation fluid into the suction lumen of the aspiration catheter 150. For example, proximal end of the cutting head 200 has a diameter (A), and it may be preferable for the radius of the concave shape to be shallow or deep to make a cutting edge to on the proximal end. Alternatively, referring to FIG. 6A. the ends of the cutting surface or cutting edge 235 can be scalloped or jagged to further aid in cutting. An example of the depth of the concave proximal (or radius) end may be between 0.635 mm and 2.54 mm, such as 0.635 mm, 1.27 mm, 1.905 mm or 2.54 mm (0.025 inch and 0.100 inch, such as 0.025 inch, 0.050 inch, 0.075 inch or 0.100 inch).

The concave shape of the proximal end of the cutting head 200 has a radial interior (closest radially to the wire guide shaft 165) and a radial exterior (furthest radially from the wire guide shaft 165), wherein the radial exterior is proximal of the interior. As shown in FIG. 6, the distance or length between the most distal portion of the convex-shaped distal end 235 and the interior of the concaved-shaped proximal end 230 is identified as length (F). And the distance or length between the most distal portion of the convex-shaped distal end 235 and the exterior of the concaved-shaped proximal end 230 is identified as length (B). It may be preferable for the difference between F and B can be substantial such that edge 235 is defined and sharp to aid in cutting or coring the lesion.

Continuing to refer to FIG. 6, the convex shape of the distal end of the cutting head 200 has a diameter (E), wherein the diameter of the cutting head 200 is determined at a position where the distal end 220 of the blades 215 are located. It may be preferable for the ratio of diameter E:diameter A or etc. to be between about 1:4 to 1:1.5 such as about 1:4, 1:3.5, 1:3, 1:2.5, 1:2, 1:1.5.

It may be preferable for the ratio of length B of the cutting head 200 to the diameter A of the proximal end of the cutting head 200 or etc. to be between about 1.5: 1 and 3:1, such as about 1.5:1.0, 1.75:1.0, 2.0:1.0, 2.25:1.0, 2.50:1.0, 2.75:1.0 or 3.0:1.0.

Continuing to refer to FIG. 6, and as discussed above, the blades 215 have a length (D) that extends from the proximal end 225 to the distal end 220. It may be preferable for the percentage of length D of the blades 215 to the length B of the cutting head 200 or etc. to be a portion of length B, such as a ten percent or as much as the entire length of B where blades 215 are from cutting edge 235 to the distal tip of 200. The blades 215 can converge at the distal end of 200 and can be in communication or meet at the distal end. As shown in FIG. 6, the proximal end 225 of the blade 215 is located distally of the concave proximal end of the cutting head 200, and the distal end 220 of the cutting head 200 is located proximally of the convex distal end of the cutting head 200. However, the proximal end 225 of the blade 215 can begin at the concave proximal end of the cutting head 200 and extend to the convex distal end of the cutting head 200 or a location proximal of the convex distal end of the cutting head 200. Also, the distal end 220 of the blade 215 can begin at the convex distal end of the cutting head 200 and extend to the concave proximal end of the cutting head 200 or a location distal of the concave proximal end of the cutting head 200.

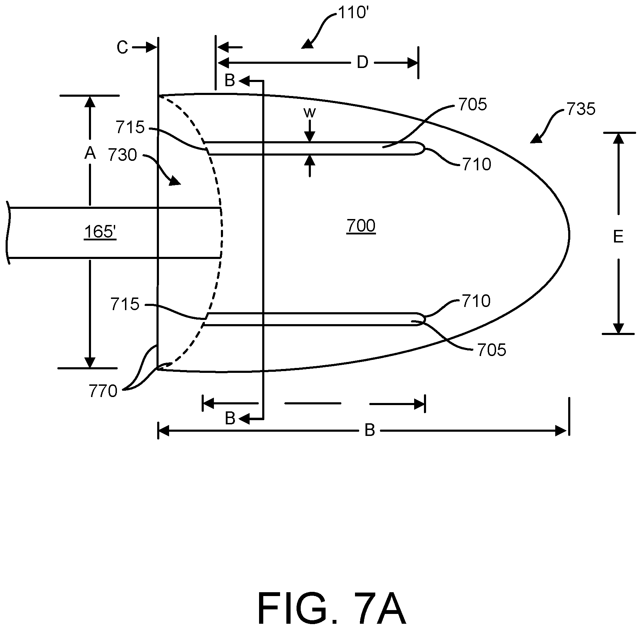

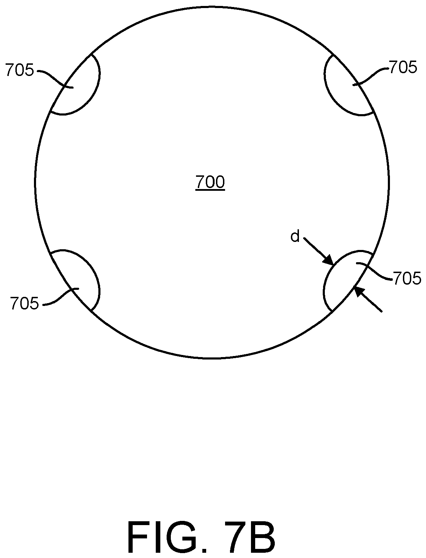

Referring to FIG. 7A and FIG. 7B, there is shown an alternative non-rotatable wire guide 110', including wire guide shaft 165' and cutting head 700, wherein the cutting head 700 has a concave-shaped proximal end 730 and a convex-shaped distal end 735. In comparison to the cutting head 200 illustrated in FIGs. 5, 6 and 6A, which has a plurality of blades 215 extending from the exterior of the cutting head 200, the cutting head 700 includes a plurality of grooves 705 that are recessed within the exterior of the cutting head 700. Each groove 705 has a distal end 710 and a proximal end 715, and the grooves 705 are evenly spaced around the exterior or circumference of the cutting head 700 and are aligned with and parallel to the longitudinal axis of the non-rotatable wire guide 110' and cutting head 700. Since each groove 705 is recessed within the cutting head 700, each groove 705 has two sharp cutting edges 220 on each side of the groove 705.

Each of the grooves 705 has a depth (d) extending from the surface of the cutting head 700 toward its center. The depth (d) of each or some of the grooves 705 may be constant from the distal end 710 to the proximal end 715, the depth (d) of each or some of the grooves 705 may increase as the groove progresses from the distal end 710 to the proximal end 715, or the depth (d) of each or some of the grooves 705 may decrease as the groove progresses from the distal end 710 to the proximal end 715. The configuration(s) of the height of the grooves 705 may increase the ability and effectiveness of the grooves 705 to cut through the chronic clot or thrombus.

The width (w) of each or some of the grooves 705 may be constant from the distal end 710 to the proximal end 715, the width (w) of each or some of the grooves 705 may increase as the groove progresses from the distal end 710 to the proximal end 715, or the width (w) of each or some of the grooves 705 may decrease as the groove progresses from the distal end 710 to the proximal end 715. The configuration(s) of the width of the grooves 705 may increase the ability and effectiveness of the grooves 705 to cut through the chronic clot or thrombus.

Continuing to refer to FIG. 7A and FIG. 7B, there are shown reference items A, B, C, E and F of cutting head 700 which are the same or similar to the reference items A, B, C, E and F of cutting head 200 illustrated in FIG. 6. Rather than having a plurality of blades 215 extending from the exterior of the cutting head 200, the cutting head 700 includes a plurality of grooves 705 that are recessed within the exterior of the cutting head 700. Nevertheless, the ratios that include and/or relate to reference items A, B, C, E and F of cutting head 200 in FIG. 6 apply to the reference items A, B, C, E and F of cutting head 700 depicted in FIGs. 7A and 7B.

Continuing to refer to FIGs. 7A and 7B, the grooves have a length (D) that extends from the proximal end 715 to the distal end 710. It may be preferable for the percentage of length D of the grooves 705 to the length B of the cutting head 700 or etc. to be a small as ten percent of the length of 700 or full length of 700. Grooves 705 can converge at the distal end of 110 such that the grooves communicate or end at the distal tip. As shown in FIGs. 7A and 7B, the proximal end 715 of the groove 705 is located at the concave proximal end of the cutting head 700, and the distal end 710 of the cutting head 700 is located proximally of the convex distal end of the cutting head 700. However, the proximal end 715 of the groove 705 can begin distal of the concave proximal end of the cutting head 700 and extend to the convex distal end of the cutting head 700 or a location proximal of the convex distal end of the cutting head 700. Also, the distal end 220 of the groove 705 can begin at the convex distal end of the cutting head 200 and extend to the concave proximal end of the cutting head 700 or a location distal of the concave proximal end of the cutting head 700.

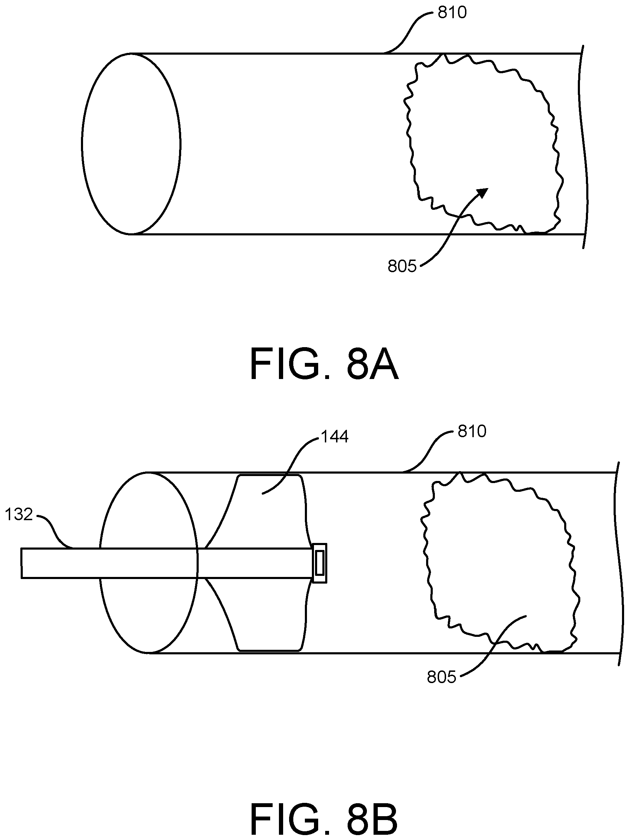

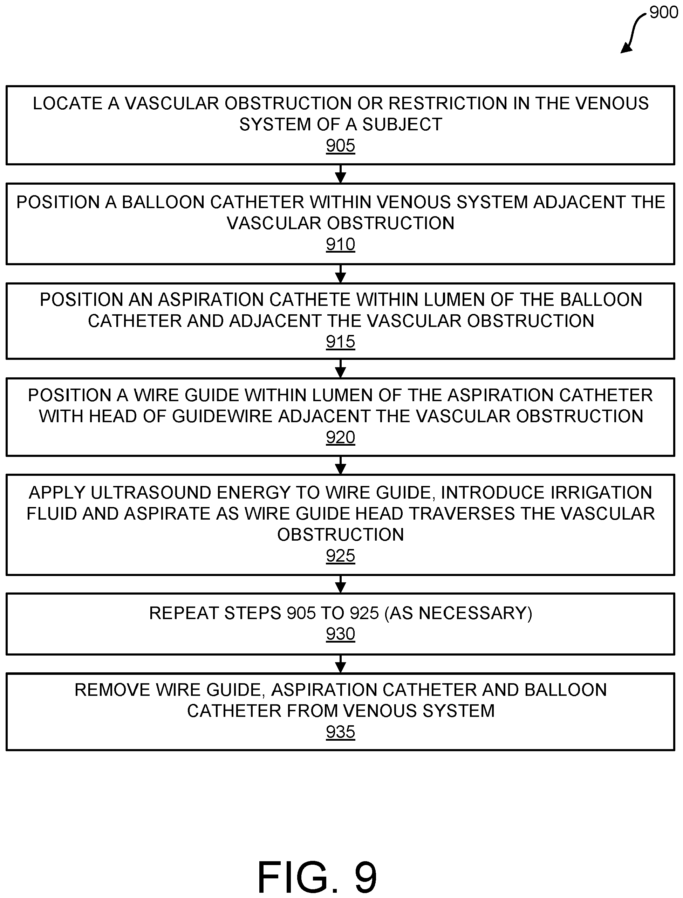

Referring to FIG. 9, there is depicted a flow chart illustrating the steps of an example method 900 of using, for example, the kit 190 (depicted in FIG. 2) that includes the non-rotatable wire guide 110, the balloon catheter assembly 130 (depicted in FIG. 3) and the aspiration catheter assembly 150 (depicted in FIG. 4) to create a lumen in and through the thrombus or chronic clot or vascular obstruction in a human subject's vascular system, particularly the subjects venous system. For example, FIG. 8A depicts a chronic clot 805 within a vein 810. Method 900 in FIG. 9 includes locating the chronic clot 805 in the vein of a subject at step 905. The next step 910 of the method 900 includes positioning a balloon catheter 130 (e.g., sheath 132) within the vein 810 of the subject adjacent the chronic clot 805, wherein the balloon catheter 130 comprises an expandable member 144. Upon positioning the balloon catheter 130 (e.g., sheath 132) adjacent the chronic clot, the expandable member 144 is expanded within the vein 810 as shown in FIG. 8B typically with a contrast and saline solution for visibility under x-ray/fluoroscopy.

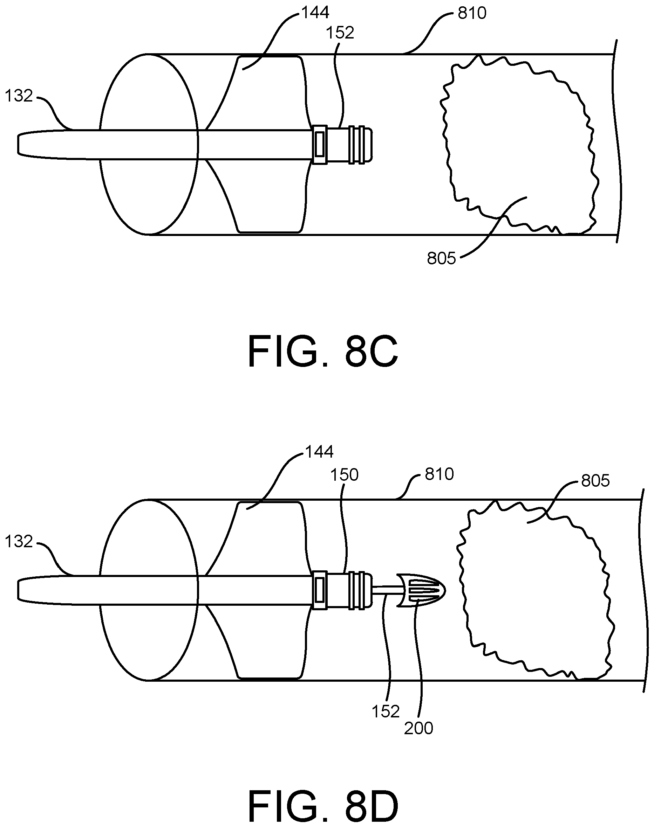

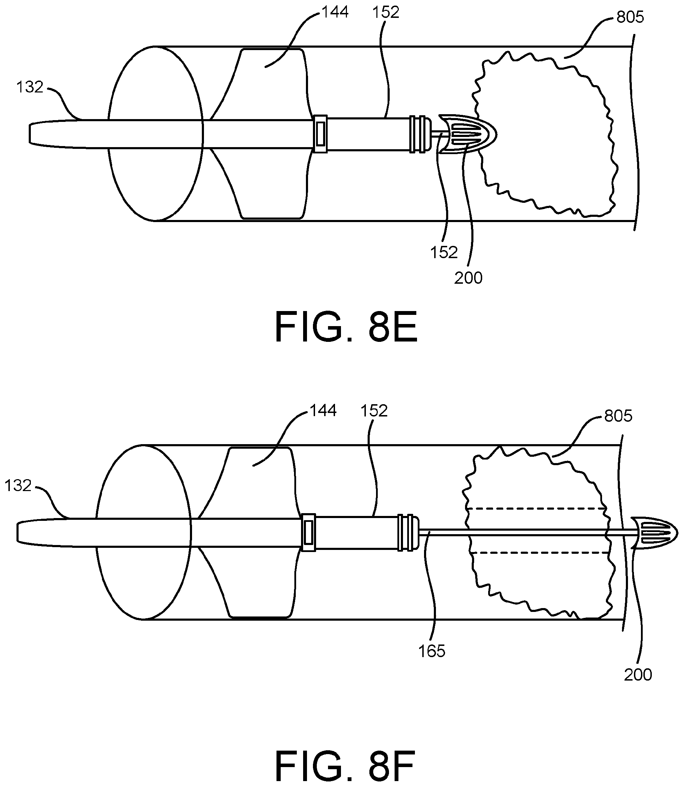

Referring again to FIG. 9, the method 900 also includes the step 915 of positioning an aspiration catheter 150 within the vein 810 of the subject, wherein the aspiration catheter 150 (e.g., sheath 152) extends through a lumen of the balloon catheter 130 (e.g., sheath 132) beyond a distal end of the balloon catheter 130 (e.g., sheath 132) and adjacent the chronic clot 805, as shown in FIG. 8C. The next step 920 of the method 900 includes positioning a non-rotatable wire guide 110 within the vein 810 of the subject, wherein the non-rotatable wire guide 110 (including the wire guide shaft 165 and the cutting head 200) extends through a lumen of the aspiration catheter 150 (e.g., sheath 152) beyond a distal end of the aspiration catheter 150 (e.g., sheath 132) and adjacent the chronic clot 805, as shown in FIG. 8D. The next step 920 of the method 900 includes introducing an irrigation fluid, such as sterile saline, into the working channel of the aspiration catheter 150 such that the irrigation fluid reaches the chronic clot 805 and simultaneously applying ultrasonic energy to the non-rotatable wire guide 110 such that the wire guide shaft 165 and the cutting head 200 of the non-rotatable wire guide 110 translates back and forth axially without rotating as wire guide shaft 165 and the cutting head 200 traverse the chronic clot, as shown in FIGs. 8E and 8F. The irrigation fluid in the aspiration catheter or irrigation sheath also assists in cooling the wire guide 110 as the wire guide 110 vibrates and/or or creates friction with the lesion. As the cutting head 200 traverses the chronic clot 805, the aspiration catheter 150 (e.g., sheath 152) aspirates the irrigation fluid during translation of the cutting head 200 through the chronic clot. The user of the kit 190 can repeat any of the steps 905 through 925, as necessary. After completion of steps 905 to 930, the non-rotatable wire guide, the aspiration catheter and the balloon catheter (after deflation of the expandable member) are removed from the vascular system.

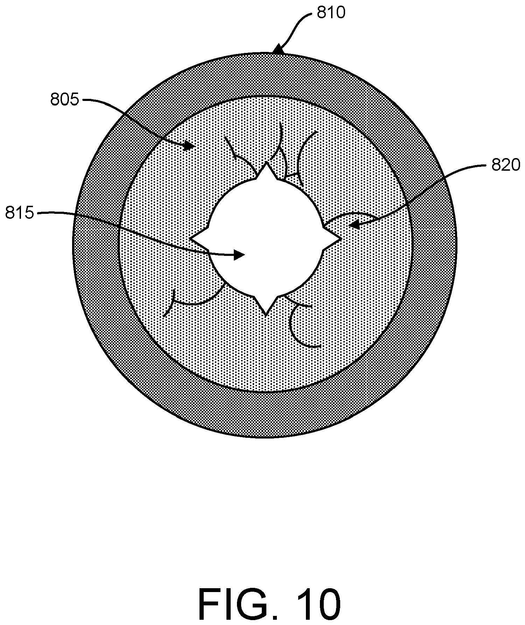

Referring to FIG. 10, upon completion of the method 900, a lumen 815 is cored or created through the thrombus or chronic clot or vascular obstruction 805 within the subject's vasculature 820. As shown in this figure, the indentations 820 within the vascular obstruction correspond to the shape of the blades 215 on the cutting head 200.

The present disclosure, in various aspects, embodiments, and configurations, includes components, processes, systems and/or apparatus substantially as depicted and described herein, including various aspects, embodiments, configurations, sub combinations, and subsets thereof. Methods for using the systems are exemplarily described. Those of skill in the art will understand how to make and use the various aspects, aspects, embodiments, and configurations, after understanding the present disclosure. The present disclosure, in various aspects, embodiments, and configurations, includes providing devices and processes in the absence of items not depicted and/or described herein or in various aspects, embodiments, and configurations hereof, including in the absence of such items as may have been used in previous devices or processes, for example, for improving performance, achieving ease and/or reducing cost of implementation.

The foregoing discussion of the disclosure has been presented for purposes of illustration and description. In the foregoing Detailed Description for example, various features of the disclosure are grouped together in one or more, aspects, embodiments, and configurations for the purpose of streamlining the disclosure. The features of the aspects, embodiments, and configurations of the disclosure may be combined in alternate aspects, embodiments, and configurations other than those discussed above. This method of disclosure is not to be interpreted as reflecting an intention that the claimed disclosure requires more features than are expressly recited in each claim. Rather, as the following claims reflect, inventive aspects lie in less than all features of a single foregoing disclosed aspects, embodiments, and configurations. Thus, the following claims are hereby incorporated into this Detailed Description, with each claim standing on its own as a separate preferred embodiment of the disclosure. The invention is defined by the following claims.

## Claims

1. A catheter system comprising:
a balloon catheter (130) comprising an expandable member (144);
an aspiration catheter (150) configured to extend beyond a distal end of the balloon catheter; and
a non-rotatable wire guide (110) comprising a cutting head (200), wherein the cutting head comprises a proximal end and a distal end, wherein the proximal end comprises a concave shape and the distal end comprises a convex shape, wherein the concave shape comprises a most proximal end and a most distal end, wherein the proximal end of the cutting head comprises a diameter, wherein a ratio of a length between the distal end of the cutting head and the most proximal end of the concave shape relative to the diameter of the cutting head is between 1.5:1 and 3:1, wherein the non-rotatable wire guide such is configured to translate back and forth axially without rotating;
wherein the non-rotatable wire guide is configured to be connected to an ultrasonic generator to apply ultrasonic energy to the non-rotatable wire guide such that the cutting head of the non-rotatable wire guide translates back and forth axially without rotating.

2. The catheter system of claim 1, wherein the cutting head comprises a plurality of blades (215) evenly spaced around a circumference of the cutting head.

3. The catheter system of claim 2, wherein the blades are substantially parallel to and aligned with a longitudinal axis of the shaft (165).

4. The catheter system of claim 2, wherein the blades have a proximal end (225), a distal end (220), a height (h) and a width (w).

5. The catheter system of claim 4, wherein the height of at least one of the plurality of blades increases from the distal end to the proximal end of the at least one of the plurality of blades.

6. The catheter system of claim 4, wherein the width of at least one of the plurality of blades increases from the distal end to the proximal end of the at least one of the plurality of blades.

7. The catheter system of claim 6, wherein the width of at least one of the plurality of blades increases from the distal end to the proximal end of the at least one of the plurality of blades.

8. The catheter system of claim 1, wherein the balloon catheter is configured to be positioned within a venous system of a subject adjacent a located obstruction, wherein the expandable member is configured to be expanded within the venous system;
wherein the aspiration catheter is configured to be positioned within the vasculature of the subject;
wherein the non-rotatable wire guide is configured to be positioned within the venous system of the subject, wherein the cutting head is configured to be positioned adjacent the chronic clot;
wherein the balloon catheter or the aspiration catheter is configured to introduce a fluid into the balloon catheter or the aspiration catheter; and
wherein the non-rotatable wire guide is configured to aspirate the fluid during translation of the cutting head.

9. The catheter system of claim 8, wherein the cutting head is configured to be repositioned.

10. The catheter system of claim 8, wherein the aspiration catheter is configured to aspirate a fluid.

## Patentansprüche

1. Kathetersystem, umfassend:
einen Ballonkatheter (130), der ein ausdehnbares Element (144) umfasst;
einen Aspirationskatheter (150), der so konfiguriert ist, dass er sich über ein distales Ende des Ballonkatheters hinaus erstreckt; und
eine nicht drehbare Drahtführung (110), die einen Schneidkopf (200) umfasst, wobei der Schneidkopf ein proximales Ende und ein distales Ende umfasst, wobei das proximale Ende eine konkave Form aufweist und das distale Ende eine konvexe Form aufweist, wobei die konkave Form ein am weitesten proximales Ende und ein am weitesten distales Ende umfasst, wobei das proximale Ende des Schneidkopfes einen Durchmesser umfasst, wobei ein Verhältnis einer Länge zwischen dem distalen Ende des Schneidkopfes und dem am weitesten proximalen Ende der konkaven Form relativ zum Durchmesser des Schneidkopfes zwischen 1,5:1 und 3:1 liegt, wobei die nicht drehbare Drahtführung so konfiguriert ist, dass sie sich axial hin und her verschiebt, ohne sich zu drehen;
wobei die nicht drehbare Drahtführung so konfiguriert ist, dass sie mit einem Ultraschallgenerator verbunden werden kann, um Ultraschallenergie auf die nicht drehbare Drahtführung anzuwenden, sodass sich der Schneidkopf der nicht drehbaren Drahtführung axial hin und her verschiebt, ohne sich zu drehen.

2. Kathetersystem nach Anspruch 1, wobei der Schneidkopf eine Vielzahl von Klingen (215) umfasst, die gleichmäßig um einen Umfang des Schneidkopfs verteilt sind.

3. Kathetersystem nach Anspruch 2, wobei die Klingen im Wesentlichen parallel zu einer Längsachse des Schafts (165) verlaufen und mit dieser ausgerichtet sind.

4. Kathetersystem nach Anspruch 2, wobei die Klingen ein proximales Ende (225), ein distales Ende (220), eine Höhe (h) und eine Breite (w) aufweisen.

5. Kathetersystem nach Anspruch 4, wobei die Höhe mindestens einer der Vielzahl von Klingen vom distalen Ende zum proximalen Ende der mindestens einen der Vielzahl von Klingen zunimmt.

6. Kathetersystem nach Anspruch 4, wobei die Breite mindestens einer der Vielzahl von Klingen vom distalen Ende zum proximalen Ende der mindestens einen der Vielzahl von Klingen zunimmt.

7. Kathetersystem nach Anspruch 6, wobei die Breite mindestens einer der Vielzahl von Klingen vom distalen Ende zum proximalen Ende der mindestens einen der Vielzahl von Klingen zunimmt.

8. Kathetersystem nach Anspruch 1, wobei der Ballonkatheter so konfiguriert ist, dass er innerhalb eines Venensystems eines Subjekts positioniert werden kann, das sich neben einer lokalisierten Verstopfung befindet, wobei das ausdehnbare Element so konfiguriert ist, dass es innerhalb des Venensystems ausgedehnt werden kann;
wobei der Aspirationskatheter so konfiguriert ist, dass er im Gefäßsystem des Subjekts positioniert werden kann;
wobei die nicht drehbare Drahtführung so konfiguriert ist, dass sie innerhalb des Venensystems des Subjekts positioniert werden kann, wobei der Schneidkopf so konfiguriert ist, dass er neben dem chronischen Gerinnsel positioniert werden kann;
wobei der Ballonkatheter oder der Aspirationskatheter konfiguriert ist, um eine Flüssigkeit in den Ballonkatheter oder den Aspirationskatheter einzuführen; und
wobei die nicht drehbare Drahtführung so konfiguriert ist, dass sie die Flüssigkeit während der Translation des Schneidkopfes ansaugt.

9. Kathetersystem nach Anspruch 8, wobei der Schneidkopf zur Neupositionierung konfiguriert ist.

10. Kathetersystem nach Anspruch 8, wobei der Aspirationskatheter zum Absaugen einer Flüssigkeit konfiguriert ist.

## Revendications

1. Système de cathéter comprenant:
un cathéter à ballonnet (130) comprenant un élément extensible (144);
un cathéter d'aspiration (150) configuré pour s'étendre au-delà d'une extrémité distale du cathéter à ballonnet; et
un guide de fil non rotatif (110) comprenant une tête de coupe (200), où la tête de coupe comprend une extrémité proximale et une extrémité distale, où l'extrémité proximale comprend une forme concave et l'extrémité distale comprend une forme convexe, où la forme concave comprend une extrémité la plus proximale et une extrémité la plus distale, où l'extrémité proximale de la tête de coupe comprend un diamètre, où le rapport d'une longueur entre l'extrémité distale de la tête de coupe et l'extrémité la plus proximale de la forme concave par rapport au diamètre de la tête de coupe est compris entre 1,5:1 et 3:1, où le guide de fil non rotatif est configuré pour effectuer une translation d'avant en arrière axialement sans tourner;
où le guide de fil non rotatif est configuré pour être connecté à un générateur ultrasonique afin d'appliquer une énergie ultrasonique au guide de fil non rotatif de telle sorte que la tête de coupe du guide de fil non rotatif effectue une translation axiale d'avant en arrière sans tourner.

2. Système de cathéter selon la revendication 1, dans lequel la tête de coupe comprend une pluralité de lames (215) uniformément espacées autour d'une circonférence de la tête de coupe.

3. Système de cathéter selon la revendication 2, dans lequel les lames sont sensiblement parallèles et alignées avec un axe longitudinal de la tige (165).

4. Système de cathéter selon la revendication 2, dans lequel les lames ont une extrémité proximale (225), une extrémité distale (220), une hauteur (h) et une largeur (w).

5. Système de cathéter selon la revendication 4, dans lequel la hauteur d'au moins une de la pluralité de lames augmente depuis l'extrémité distale jusqu'à l'extrémité proximale de l'au moins une de la pluralité de lames.

6. Système de cathéter selon la revendication 4, dans lequel la largeur d'au moins une de la pluralité de lames augmente depuis l'extrémité distale jusqu'à l'extrémité proximale de l'au moins une de la pluralité de lames.

7. Système de cathéter selon la revendication 6, dans lequel la largeur d'au moins une de la pluralité de lames augmente depuis l'extrémité distale jusqu'à l'extrémité proximale de l'au moins une de la pluralité de lames.

8. Système de cathéter selon la revendication 1, dans lequel le cathéter à ballonnet est configuré pour être positionné à l'intérieur d'un système veineux d'un sujet adjacent à une obstruction localisée, où l'élément extensible est configuré pour être étendu à l'intérieur du système veineux;
où le cathéter d'aspiration est configuré pour être positionné à l'intérieur du système vasculaire du sujet;
où le guide de fil non rotatif est configuré pour être positionné à l'intérieur du système veineux du sujet, où la tête de coupe est configurée pour être positionnée à proximité du caillot chronique;
où le cathéter à ballonnet ou le cathéter d'aspiration est configuré pour introduire un fluide dans le cathéter à ballonnet ou dans le cathéter d'aspiration; et
où le guide de fil non rotatif est configuré pour aspirer le fluide pendant la translation de la tête de coupe.

9. Système de cathéter selon la revendication 8, dans lequel la tête de coupe est configurée pour être repositionnée.

10. Système de cathéter selon la revendication 8, dans lequel le cathéter d'aspiration est configuré pour aspirer un fluide.
